# EUROPEAN PATENT SPECIFICATION

(11) **EP 4 201 482 B1**
(45) Date of publication and mention of the grant of the patent: **07.10.2026**
(21) Application number: 21865506.6
(22) Date of filing: 24.03.2021
(51) Int. Cl.: A61N 7/02, A61B 18/12, A61B 18/00, A61B 34/10, A61B 90/00

(54) **AUTOMATIC PLANNING METHOD AND DEVICE FOR TISSUE ABLATION**
AUTOMATISCHES PLANUNGSVERFAHREN UND VORRICHTUNG ZUR GEWEBEABLATION
PROCÉDÉ ET DISPOSITIF DE PLANIFICATION AUTOMATIQUE POUR ABLATION DE TISSU

(30) Priority: 09.09.2020 CN 202010941482
(43) Date of publication of application: 28.06.2023
(73) Proprietor: Shanghai Shende Green Medical Era Healthcare Technology Co., Ltd., Shanghai 200233 (CN); Shende (Ningbo) Medical Device Technology Co., Ltd, Ningbo, Zhejiang 315800 (CN); Nantong Shende Medical Device Technology Co., Ltd, Nantong, Jiangsu 226014 (CN)
(72) Inventor: GUO, Junjie, Shanghai 200233 (CN); ZHANG, Shengfa, Shanghai 200233 (CN); WEI, Bo, Shanghai 200233 (CN); YANG, Bo, Shanghai 200233 (CN); GU, Jiawei, Shanghai 200233 (CN); WU, Hao, Shanghai 200233 (CN); LIU, Zihao, Shanghai 200233 (CN); SU, Zhiqiang, Shanghai 200233 (CN); WEN, Jiabao, Shanghai 200233 (CN)
(74) Representative: Osterhoff, Utz
(86) International application number: PCT/CN2021/082586
(87) International publication number: WO 2022/052443

(56) References cited:
- WO-A1-2011/080666
- CN-A- 105 534 593
- CN-A- 108 805 991
- CN-A- 112 007 289
- US-A1- 2009 118 613
- US-A1- 2012 237 105
- US-A1- 2012 237 105
- US-A1- 2017 224 402
- US-A1- 2020 179 051

## Description

### TECHNICAL FIELD

The present disclosure relates to the technical field of medical technology, in particular to an automatic planning method and a device for tissue ablation.

### BACKGROUND

Ultrasonic thermotherapy treatment of tumors is gradually and widely used clinically due to its features of non-invasive treatment. During ultrasonic treatment, it is necessary to use non-invasive monitoring technologies such as magnetic resonance and B-mode ultrasonography to image the treatment position. Imaging is used for lesion identification, sensitive tissue identification, automatic planning system for ablation area, temperature monitoring during treatment, and patient movement monitoring. In the planning of traditional ultrasonic thermotherapy, two dimensional (2D) images are used as the reference images for planning, such as magnetic resonance, ultrasonic and computed tomography (CT). During planning, doctors will add corresponding planning elements to images in different directions, including lesion area, skin, energy-blocked areas, low energy density areas, important organs, blood vessels and nerves, etc. These planning elements play a vital role in safe and effective thermotherapy for patients. Therefore, during each ultrasonic thermal ablation process, it is necessary to fully consider the impact of this thermotherapy process on the patient's important organs, blood vessels and other sensitive tissues, including whether the expected thermal effect can be accurately produced at the target point, whether it will overheat the lesion tissue, and whether it will cause unexpected damage to the patient's important organs, blood vessels or sensitive tissues, etc., if necessary, it is required to calculate the data such as the distance and the direction of the heating point relative to the patient's lesion, skin, sensitive tissue and other elements, thereby an optimal heating parameter suitable for the current equipment can be calculated to achieve the optimal thermotherapy effect. However, accurate data cannot be obtained directly from 2D imaging data. If the calculated relevant data is inaccurate, it may lead to incomplete treatment and increase the potential recurrence of tumors. In the traditional method, physicians need to rescan the internal situation of the patient's body, redepict the patient's lesions, skin, sensitive tissues and mark the ablated areas. The redepicted planning elements may deviate from the previously depicted planning elements, and the ablated area cannot be well identified on the image, which greatly tests the experience and technical level of the physician. If there is a deviation, it will lead to over-ablation or no ablation of a certain tissue, resulting in incomplete treatment and tumor recurrence.

As for the irregular shape or large lesion area, doctors must plan in advance how to prevent ablation points from affecting normal tissues, how many ablation points are needed and the order of these ablation points to avoid interference. As for the complicated operation, doctors must use cylindrical ablation volumes of different sizes and orientations on planning images to fill the complete range of the three dimensional (3D) tumor volume. In the planning process, inaccurate or insufficient planning will lead to incomplete treatment and potential recurrence of the tumor. Traditional methods plan in a point-by-point manner, but will greatly lengthen the process time and make the operation difficult.

US 2012/237105 A1 discloses an ablation treatment planning and, optionally, guiding method usable for e.g. tumour tissue ablation with a cryoablation needle which cools down an adjacent tumour tissue to thereby generate an ablation volume is proposed. In order to be able to plan an ablation treatment, a 3D image data set of a region of interest may be acquired by e.g. X-ray imaging using e.g. a C-arm system. Then, 3D model data of the ablation volume are introduced into the 3D image data set for example by tagging image pixels using a stencil buffer and possibly by culling specific inside areas and/or outside areas of the ablation volume. Finally, a 2D image to be visualized to a physician and comprising a projection of the region of interest and the ablation volume is drawn wherein an MPR (multi planar reformatting) plane in which the 2D image is drawn is used as a clipping plane. With such graphical approach, an ablation volume having any arbitrary shape such as for example an ellipsoid shape may be visualized within a 3D image space by drawing 2D images in any desired MPR plane such that also oblique orientations of the ablation volume can be represented. In a subsequent guiding procedure, an ablation needle may be guided to a location and in an orientation as previously planned.

US 2009/118613 A1 discloses a method for determining a volume of ablated tissue includes the steps of supplying energy to tissue, indicating an axis within the tissue, and simulating slicing of the tissue substantially perpendicular to the axis to obtain a plurality of simulated slices. Each of the plurality of simulated slices has a thickness, a cross-sectional perimeter, and a trajectory point defined by the axis within the tissue. The method further includes the steps of determining a volume of each of the plurality of simulated slices based on the trajectory point, the cross-sectional perimeter, and the thickness of each simulated slice, and summing the volumes from each of the plurality of simulated slices to obtain the volume of the ablated tissue.

### SUMMARY

The invention is defined in independent claims 1 and 9. Certain optional features of the invention are defined in the dependent claims. The main objective of the present disclosure is to provide an automatic planning method and a device for tissue ablation in order to overcome the above-mentioned defects in the prior art, which has a higher automation, higher precision and good safety.

One objective of the present disclosure can be achieved through the following technical solutions:
an automatic planning method for tissue ablation, including:
obtaining a 3D model of a to-be-ablated tissue through a 3D reconstruction technique;
marking a cylindrical ablation point on the 3D model through an ablation planning, an axial direction of the ablation point is the same as a radio frequency direction of thermal ablation; and
displaying an ablated area on the 3D model;
the 3D reconstruction technique includes:
   obtaining slice images of the to-be-ablated tissue in a plurality of directions, the slice image in each direction includes a plurality of 2D images;
   depicting, by a primitive, the to-be-ablated tissue on the 2D images in one direction; constructing the 3D model of the to-be-ablated tissue through the 3D reconstruction technique based on the original 2D images;
   displaying a cross section of the 3D model of the to-be-ablated tissue on the 2D image in other un-depicted directions;
   the ablation planning includes:
      selecting parameters of the ablation point and an implement sequence, the parameters of the ablation point include a size, a direction, and a frequency of the ablation point;
      filling the ablation point on the 3D model according to the implement sequence, the filling includes:
         dividing the 3D model into a plurality of ablation layers in a direction perpendicular to the radio frequency direction of thermal ablation; dividing the ablation layers into a plurality of strip-shaped first to-be-ablated blocks in the direction of radio frequency direction of thermal ablation;
         in response that there is the ablated area in the first to-be-ablated block, dividing the first to-be-ablated block into a second to-be-ablated block and an ablated block, the ablated block is located in front of the ablated area in the radio frequency direction of thermal ablation; and
         filling the ablation points of one parameter in the first to-be-ablated block and the second to-be-ablated block, which decreases heat accumulation to the to-be-ablated issue as possible, and avoids the ablated area to reach the premium ablation effect; the parameters of the ablation point further includes an overlapping rate, two adjacent ablation points are overlapped when a plurality of ablation points are provided between the first to-be-ablated block and the second to-be-ablated block.

In an embodiment, a formula for calculating a thickness D of the ablation layer is: $D = d \times N$ d is a diameter of the cylindrical ablation point, and N is the overlapping rate of the two adjacent ablation points.

In an embodiment, reobtaining a 2D image when the to-be-ablated tissue moves; comparing a previous 2D image before moving and the reobtained 2D image which are in a same direction; and transforming the 3D model and the ablated area on the 3D model together into a coordinate system of the reobtained 2D image, thus avoiding the thermal ablation differential caused by the moving of the to-be-ablated tissue, which greatly improves the efficiency and the accuracy of the ablation point planning.

In an embodiment, implementing interpolation for tissues that are missing on the 2D image after depicting the 2D image of the to-be-ablated tissue, implementing interpolation for the un-depicted 2D images according to a difference between two adjacent 2D images in the same direction, improving the accuracy of depicting the primitives, further improving the accuracy of the 3D model, thus the accuracy of the ablation point planning is high.

In an embodiment, setting a loss function before constructing the 3D model, and fine-tuning the primitive on the 2D image by the loss function, a formula of the loss function *Loss* is: $Loss = 1 - \left({S}_{1}{W}_{1}+{S}_{2}{W}_{2}\right)$

S₁ is a similarity of a gray gradient and a gray mean value of edges of primitives on the two adjacent 2D images, S₂ is a similarity of primitives on the two adjacent 2D images, *W₁* and *W*₂ are preset weights, fine-tuning the image until the loss function value is smaller than the preset threshold, the fine-tuning includes expansion, erosion and vertex offset, which decrease the differential between the 3D model and the actual to-be-ablated issue, and improves the accuracy of the thermal ablation.

In an embodiment, an automatic planning device is provided, including: a memory and a processor, a computer program is stored in the memory, and the computer program is invoked by the processor to execute the above methods.

Compared with the prior art, the present disclosure has the following beneficial effects.
(1) The present disclosure depicts primitives on a plane image in a direction of a to-be-ablated tissue, obtains a 3D model through 3D reconstruction technique, and displays the ablated area on the 3D model simultaneously, thus avoiding excessive ablation of the tissue after multiple times of thermal ablation, and is of good safety. By providing ablation points and application sequences with a plurality of parameters, the 3D model is divided into ablation layers, and then the ablation layer is divided into long strips of the first to-be-ablated block, if an ablated area is in the first to-be-ablated blocks, dividing the first to-be-ablated block into a second to-be-ablated block and an ablated block; and filling the ablation points of one parameter in the first to-be-ablated block and the second to-be-ablated block in the radio frequency direction of thermal ablation, so that the ablation points cover the to-be-ablated tissue to the greatest extent, with high automation and high precision.
(2) The present disclosure allows two adjacent ablation points to partially overlap when filling the ablation point, to avoid no ablation and improve the accuracy of planning ablation point.
(3) The present disclosure reobtains a 2D image when the to-be-ablated tissue moves, and compares a previous 2D image before moving and the reobtained 2D image which are in a same direction, transforms the 3D model and the ablated area on the 3D model together into a coordinate system of the reobtained 2D image, to avoid the error of thermal ablation caused by the movement of the ablated tissue, and improve the efficiency and accuracy.
(4) The present disclosure complements the un-depicted 2D images according to a difference between two adjacent 2D images in the same direction, and complements tissues that are missing on the 2D image after depicting the 2D image of the to-be-ablated tissue, to improve the accuracy of depicting primitives, to further improve the accuracy of the 3D model and the accuracy of planning ablation points.
(5) Before constructing the 3D model, the present disclosure establishes a loss function based on the similarity of the primitives on two adjacent 2D images and the similarity of a gray gradient and a gray mean value of edges of primitives on the two adjacent 2D images. The image fine-tuning includes expansion, erosion and vertex offset. The error between the 3D model and the actual to-be-ablated tissue is reduced, to improve the accuracy of thermal ablation.

### BRIEF DESCRIPTION OF THE DRAWINGS

FIG. 1 is a method flowchart of the present disclosure.
FIG. 2 is a plan view of an ablation layer before filling an ablation point.
FIG. 3 is a plan view of the ablation layer after filling the ablation point.
FIG. 4 is a perspective view of the ablation point.
FIG. 5 is a front view of the ablation point.
FIG. 6 is a method flowchart of 3D reconstruction technique.
FIG. 7 is another method flowchart of 3D reconstruction technique.

### DETAILED DESCRIPTION OF THE EMBODIMENTS

The technical solutions of the embodiments of the present disclosure will be described in more detail below with reference to the accompanying drawings. It is obvious that the embodiments to be described are only some rather than all of the embodiments of the present disclosure. All other embodiments obtained by those skilled in the art based on the embodiments of the present disclosure without creative efforts shall fall within the scope of the present disclosure.

### First embodiment

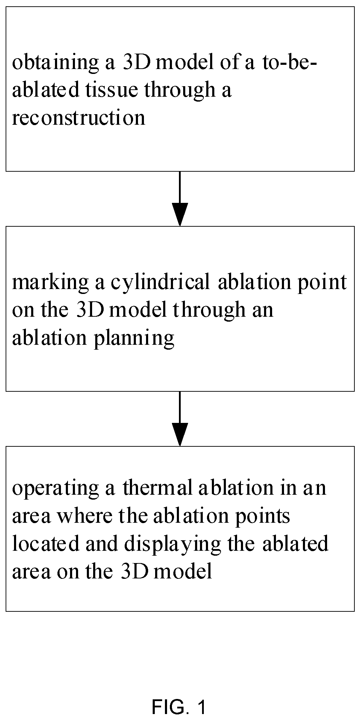

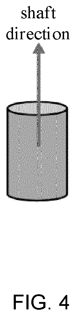

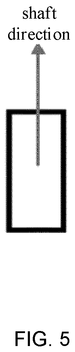

An automatic planning method for tissue ablation, as shown in FIG. 1, FIG. 4 and FIG. 5, includes:
obtaining a 3D model of a to-be-ablated tissue through a 3D reconstruction technique, the to-be-ablated tissue includes lesion, skin and visceral organ; marking a cylindrical ablation point on the 3D model through an ablation planning, an axial direction of the ablation point is the same as a radio frequency direction of thermal ablation; and displaying an ablated area on the 3D model.

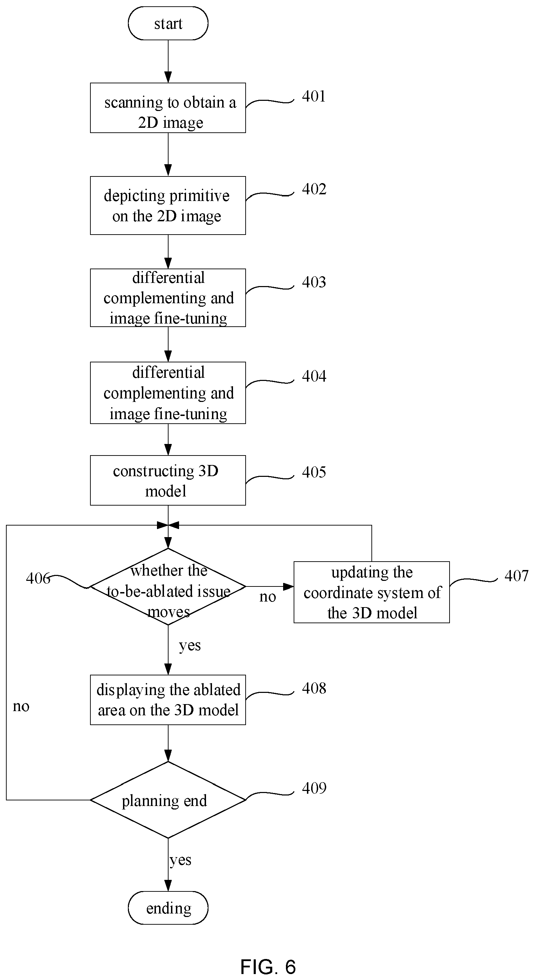

As shown in FIG. 6, the 3D reconstructions technique include:
401, obtaining slice images of the to-be-ablated tissue in a plurality of directions, the slice image in each direction comprises a plurality of 2D images;
402, depicting, by a primitive, the to-be-ablated tissue on the 2D images in one of the plurality of directions by primitive including closed polygons and polylines;
403, implementing interpolation for the missing tissue on the depicted 2D images, implementing interpolation for the un-depicted 2D images based on the adjacent 2D image in the same direction;
404, using the loss function to perform image fine-tuning on the primitive on the 2D image. The image fine-tuning includes expansion, erosion and vertex offset. The calculation formula *Loss* of the loss function is: $Loss = 1 - \left({S}_{1}{W}_{1}+{S}_{2}{W}_{2}\right)$
S₁ is the similarity between the gray gradient and the gray mean value of the primitive on two adjacent 2D images, S₂ is the similarity between the primitive on two adjacent 2D images, W₁ and W₂ are the preset weights;
405, constructing a 3D model of the to-be-ablated tissue through ITK registration technique based on the original 2D image, and displaying a cross section of the 3D model of the to-be-ablated tissue on the 2D image in other un-depicted directions; 406, determining whether the to-be-ablated tissue moves, if so, executing 407, otherwise executing 408;
407, reobtaining the 2D image, comparing the two 2D images in the same direction before and after the movement, transforming the 3D model and the ablated area on the 3D model into the coordinate system of the 2D image after the movement, and executing 406;
408, updating the ablated area on the 3D model;
409, determining whether the thermal ablation is complemented according to the ablated area on the 3D model, and if so, the planning is ended; otherwise, executing 406.

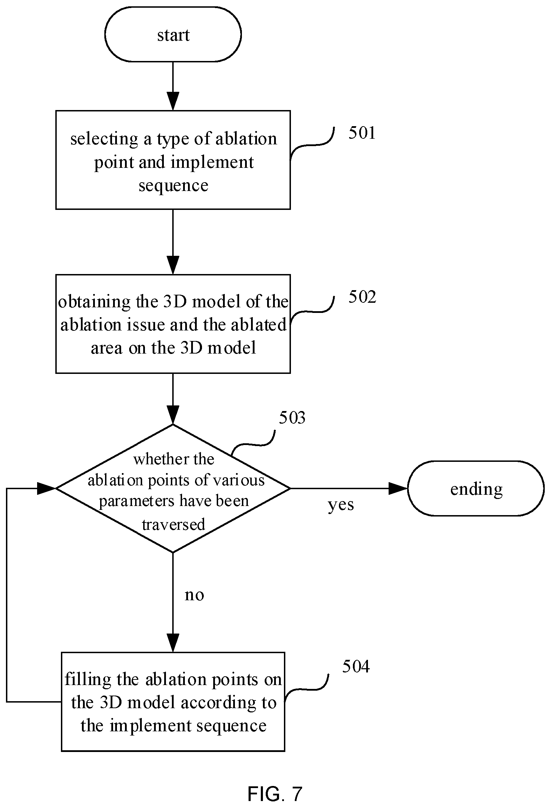

As shown in FIG. 7, the ablation planning including:
501, selecting parameters and the application sequence of the ablation point on the GUI interface, the parameters of the ablation point include a size, a direction, a frequency and an overlapping rate of the ablation point;
502, obtaining a 3D model of the to-be-ablated tissue and an ablated area on the 3D model;
503, determining whether the ablation points of various parameters are traversed, and if so, ending, otherwise executing 504;
504, filling the ablation points on the 3D model according to the application sequence, and executing 503.

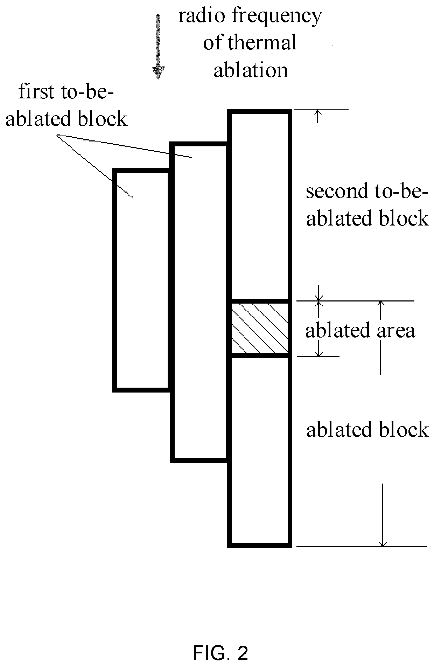

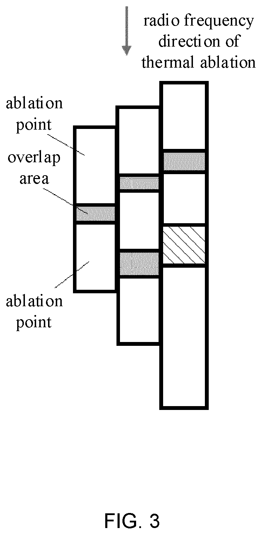

As shown in FIG. 2, FIG. 3, FIG. 4 and FIG. 5, filling the ablation points includes: dividing the 3D model into a plurality of ablation layers in the direction perpendicular to the radio frequency direction of thermal ablation, and dividing the ablation layer into a plurality of strip-shaped first to-be-ablated blocks in the direction of radio frequency direction of thermal ablation. If an ablated area is in the first to-be-ablated blocks, dividing the first to-be-ablated block into a second to-be-ablated block and an ablated block, and the ablated block is located in front of the ablated area in the radio frequency direction of thermal ablation. Based on the estimated ablation length, sequentially placing and filling the ablation points of one parameter in the first to-be-ablated block and the second to-be-ablated block in the direction of the thermal ablation radio frequency.

Two adjacent ablation points are overlapped when the first to-be-ablated block and the second to-be-ablated block are filled with a plurality of ablation points, and a formula for calculating a thickness D of the ablation layer is: $D = d \times N$ d is the diameter of the cylindrical ablation point, and N is the overlapping rate of the two adjacent ablation points.

### Second embodiment

An automatic planning device, on which an automatic planning method for tissue ablation corresponding to the first embodiment is applied, includes a memory and a processor, a computer program is stored in the memory, and the computer program is invoked by the processor to execute any method according to the first embodiment. The first embodiment and the second embodiment provide an automatic planning method and device for tissue ablation, the method includes 3D reconstructions technique and ablation planning, which can be safely and efficiently used for preoperative planning and intraoperative monitoring in the process of ultrasonic thermal ablation and postoperative evaluation, the ablation planning adopts primitive to depict the to-be-ablated tissue in the 2D image, and builds a 3D model of the to-be-ablated tissue in the 3D space, accepting the 3D input of the ablation area, when an unexpected event occurs and needs to be replanned, the 3D model and the ablated area can be updated to the new coordinate system, and the doctor does not need to manually mark the ablation point, which improves the accuracy and efficiency of ablation point planning.

**In** the description provided herein, numerous specific details are set forth. However, it is understood that embodiments of the disclosure may be practiced without these specific details. In some embodiments, well-known methods, structures and techniques have not been shown in detail in order not to obscure the understanding of this description.

It should be noted that, the above embodiments are only used to illustrate the technical solutions of the present disclosure, rather than limit the present disclosure thereto; although the present disclosure has been described in detail with reference to the above embodiments, for those skilled in the art, within the principles of the present disclosure it is still possible to modify the technical solutions described in the above embodiments, or to perform equivalent replacements for some or all of the technical features; and these modifications or replacements do not make the corresponding technical solutions deviate from the scope of the present disclosure.

## Claims

1. An automatic planning method for tissue ablation, comprising:
obtaining a three dimensional (3D) model of a to-be-ablated tissue through a 3D reconstruction technique;
marking a cylindrical ablation point on the 3D model through an ablation planning, wherein an axial direction of the ablation point is the same as a radio frequency direction of thermal ablation; and
displaying an ablated area on the 3D model;
wherein the 3D reconstruction technique comprises:
(401), obtaining slice images of the to-be-ablated tissue in a plurality of directions, wherein the slice image in each direction comprises a plurality of two dimensional (2D) images;
(402), depicting, by a primitive, the to-be-ablated tissue on the 2D images in one direction;
(405), constructing the 3D model of the to-be-ablated tissue through the 3D reconstruction technique based the original 2D images; and displaying a cross section of the 3D model of the to-be-ablated tissue on the 2D image in other un-depicted directions;
wherein the ablation planning comprises:
(501), selecting parameters of the ablation point and an implement sequence, wherein the parameters of the ablation point comprise a size, a direction, and a frequency of the ablation point;
(504), filling the ablation point on the 3D model according to the implement sequence, wherein the filling comprises:
dividing the 3D model into a plurality of ablation layers in a direction perpendicular to the radio frequency direction of thermal ablation;
dividing the ablation layers into a plurality of strip-shaped first to-be-ablated blocks in the direction of radio frequency direction of thermal ablation;
in response that there is the ablated area in the first to-be-ablated block, dividing the first to-be-ablated block into a second to-be-ablated block and an ablated block, wherein the ablated block is located in front of the ablated area in the radio frequency direction of thermal ablation; and
filling the ablation points of one parameter in the first to-be-ablated block and the second to-be-ablated block;
**characterized in that** the parameters of the ablation point further comprise an overlapping rate, two adjacent ablation points are overlapped when a plurality of ablation points are provided between the first to-be-ablated block and the second to-be-ablated block.

2. The automatic planning method for tissue ablation according to claim 1, wherein a formula for calculating a thickness D of the ablation layer is: $D = d \times N$
wherein d is a diameter of the cylindrical ablation point, and N is the overlapping rate of the two adjacent ablation points.

3. The automatic planning method for tissue ablation according to claim 1, further comprising:
reobtaining a 2D image when the to-be-ablated tissue moves;
comparing a previous 2D image before moving and the reobtained 2D image which are in a same direction; and
transforming the 3D model and the ablated area on the 3D model together into a coordinate system of the reobtained 2D image.

4. The automatic planning method for tissue ablation according to claim 1, further comprising:
implementing interpolation for the un-depicted 2D images based on the adjacent 2D image in the same direction.

5. The automatic planning method for tissue ablation according to claim 1, further comprising:
(404), setting a loss function before constructing the 3D model, and fine-tuning the primitive on the 2D image by the loss function, wherein a formula of the loss function *Loss* is: $Loss = 1 - \left({S}_{1}{W}_{1}+{S}_{2}{W}_{2}\right)$
wherein S1 is a similarity of a gray gradient and a gray mean value of edges of primitives on the two adjacent 2D images, S2 is a similarity of primitives on the two adjacent 2D images, W1 and W2 are preset weights.

6. The automatic planning method for tissue ablation according to claim 5, wherein the fine-tuning comprises expansion, erosion and vertex offset.

7. The automatic planning method for tissue ablation according to claim 1, further comprising:
(403), implementing interpolation for tissues that are missing on the 2D image after depicting the 2D image of the to-be-ablated tissue.

8. The automatic planning method for tissue ablation according to claim 1, wherein the 3D reconstruction technique is an insight segmentation and registration toolkit (ITK) technique.

9. An automatic planning device, **characterized by** comprising:
a memory and a processor,
wherein a computer program is stored in the memory, and the computer program is invoked by the processor to execute the automatic planning method for tissue ablation according to any of claims 1 to 8.

## Patentansprüche

1. Automatisches Planungsverfahren zur Gewebeablation, umfassend:
Erhalten eines dreidimensionalen (3D) Modells eines zu abladierenden Gewebes mittels einer 3D-Rekonstruktionstechnik;
Markieren eines zylindrischen Ablationspunkts auf dem 3D-Modell mittels einer Ablationsplanung, wobei eine axiale Richtung des Ablationspunkts mit einer Radiofrequenzrichtung der thermischen Ablation übereinstimmt; und
Anzeigen eines abladierten Bereichs auf dem 3D-Modell;
wobei die 3D-Rekonstruktionstechnik umfasst:
(401) Erhalten von Schnittbildern des zu abladierenden Gewebes in mehreren Richtungen, wobei das Schnittbild in jeder Richtung mehrere zweidimensionale (2D) Bilder umfasst;
(402) Einzeichnen des zu abladierenden Gewebes auf den 2D-Bildern in einer Richtung mittels eines Grafikprimitivs;
(405) Erstellen des 3D-Modells des zu abladierenden Gewebes mittels der 3D-Rekonstruktionstechnik auf Grundlage der urspr ü nglichen 2D-Bilder; und Anzeigen eines Querschnitts des 3D-Modells des zu abladierenden Gewebes auf dem jeweiligen 2D-Bild in den anderen Richtungen, in denen kein Einzeichnen vorgenommen wurde;
wobei die Ablationsplanung umfasst:
(501) Auswählen von Parametern des Ablationspunkts und einer Anwendungsreihenfolge, wobei die Parameter des Ablationspunkts eine Größe, eine Richtung und eine Frequenz des Ablationspunkts umfassen;
(504) Eintragen des Ablationspunkts in das 3D-Modell gemäß der Anwendungsreihenfolge, wobei das Eintragen umfasst:
Unterteilen des 3D-Modells in mehrere Ablationsschichten in einer Richtung senkrecht zu der Radiofrequenzrichtung der thermischen Ablation;
Unterteilen der Ablationsschichten in mehrere streifenförmige erste zu abladierende Blöcke entlang der Radiofrequenzrichtung der thermischen Ablation;
wenn sich der abladierte Bereich in dem ersten zu abladierenden Block befindet, Unterteilen des ersten zu abladierenden Blocks in einen zweiten zu abladierenden Block und einen abladierten Block, wobei sich der abladierte Block in der Radiofrequenzrichtung der thermischen Ablation vor dem abladierten Bereich befindet; und
Eintragen von Ablationspunkten mit einem bestimmten Parametersatz in den ersten zu abladierenden Block und den zweiten zu abladierenden Block;
**dadurch gekennzeichnet, dass** die Parameter des Ablationspunkts ferner eine Überlappungsrate umfassen und sich zwei benachbarte Ablationspunkte überlappen, wenn in dem ersten zu abladierenden Block und dem zweiten zu abladierenden Block mehrere Ablationspunkte vorgesehen sind.

2. Automatisches Planungsverfahren zur Gewebeablation nach Anspruch 1, wobei eine Formel zum Berechnen einer Dicke *D* der Ablationsschicht lautet: $D = d \times N$ wobei *d* ein Durchmesser des zylindrischen Ablationspunkts ist und *N* die Überlappungsrate der zwei benachbarten Ablationspunkte ist.

3. Automatisches Planungsverfahren zur Gewebeablation nach Anspruch 1, ferner umfassend:
erneutes Erhalten eines 2D-Bildes, wenn sich das zu abladierende Gewebe bewegt;
Vergleichen eines vor der Bewegung erhaltenen 2D-Bildes mit dem erneut erhaltenen 2D-Bild, wobei die beiden 2D-Bilder derselben Richtung zugeordnet sind; und
gemeinsames Transformieren des 3D-Modells und des abladierten Bereichs auf dem 3D-Modell in ein Koordinatensystem des erneut erhaltenen 2D-Bildes.

4. Automatisches Planungsverfahren zur Gewebeablation nach Anspruch 1, ferner umfassend:
Durchführen einer Interpolation für die 2D-Bilder, auf denen kein Einzeichnen vorgenommen wurde, anhand des benachbarten 2D-Bildes in derselben Richtung.

5. Automatisches Planungsverfahren zur Gewebeablation nach Anspruch 1, ferner umfassend:
(404) Festlegen einer Verlustfunktion vor dem Erstellen des 3D-Modells und Feinabstimmen des Grafikprimitivs auf dem 2D-Bild mittels der Verlustfunktion, wobei die Formel der Verlustfunktion *Loss* lautet: $Loss = 1 - \left({S}_{1}{W}_{1}+{S}_{2}{W}_{2}\right)$
wobei *S*₁ die Ähnlichkeit der Grauwertgradienten und der mittleren Grauwerte an den Rändern der Grafikprimitive zwischen den beiden benachbarten 2D-Bildern bezeichnet, *S*₂ die Ähnlichkeit der Grafikprimitive zwischen den beiden benachbarten 2D-Bildern bezeichnet und *W*₁ und *W*₂ vorgegebene Gewichtungen sind.

6. Automatisches Planungsverfahren zur Gewebeablation nach Anspruch 5, wobei die Feinabstimmung Dilatation, Erosion und Eckpunktversatz umfasst.

7. Automatisches Planungsverfahren zur Gewebeablation nach Anspruch 1, ferner umfassend:
(403) Durchführen einer Interpolation für auf dem 2D-Bild fehlende Gewebebereiche nach dem Einzeichnen des zu abladierenden Gewebes auf dem 2D-Bild.

8. Automatisches Planungsverfahren zur Gewebeablation nach Anspruch 1, wobei die 3D-Rekonstruktionstechnik eine auf dem Insight Segmentation and Registration Toolkit (ITK) basierende Technik ist.

9. Vorrichtung zur automatischen Planung, **dadurch gekennzeichnet, dass** sie Folgendes umfasst:
einen Speicher und einen Prozessor,
wobei in dem Speicher ein Computerprogramm gespeichert ist und das Computerprogramm durch den Prozessor aufgerufen wird, um das automatische Planungsverfahren zur Gewebeablation nach einem der Ansprüche 1 bis 8 auszuführen.

## Revendications

1. Procédé de planification automatique pour ablation de tissu, comprenant :
obtenir un modèle tridimensionnel (3D) d'un tissu à ablater via une technique de reconstruction 3D ;
marquer un point d'ablation cylindrique sur le modèle 3D via une planification d'ablation, une direction axiale du point d'ablation est identique à une direction de radiofréquence d'ablation thermique ; et
afficher une zone ablatée sur le modèle 3D ;
dans lequel la technique de reconstruction 3D comprend :
(401) obtenir des images en tranches du tissu à ablater dans une pluralité de directions, l'image en tranches dans chaque direction comprenant une pluralité d'images bidimensionnelles (2D) ;
(402) représenter, par un primitif, le tissu à ablater sur les images 2D dans une direction ;
(405) construire le modèle 3D du tissu à ablater via la technique de reconstruction 3D sur la base des images 2D d'origine ; et afficher une section transversale du modèle 3D du tissu à ablater sur l'image 2D dans d'autres directions non représentées,
dans lequel la planification d'ablation comprend :
(501), sélectionner des paramètres du point d'ablation et une séquence de mise en œuvre, les paramètres du point d'ablation comprenant une taille, une direction et une fréquence du point d'ablation ;
(504), remplir le point d'ablation sur le modèle 3D en fonction de la séquence de mise en œuvre, le remplissage comprenant :
diviser le modèle 3D en une pluralité de couches d'ablation dans une direction perpendiculaire à la direction de radiofréquence d'ablation thermique ;
diviser les couches d'ablation en une pluralité de premiers blocs à ablater en forme de bande dans la direction de radiofréquence d'ablation thermique ;
en réponse à ce qu'il existe la zone ablatée dans le premier bloc à ablater, diviser le premier bloc à ablater en un second bloc à ablater et un bloc ablaté, le bloc ablaté étant situé devant la zone ablatée dans la direction de radiofréquence d'ablation thermique ; et
remplir les points d'ablation d'un paramètre dans le premier bloc à ablater et le second bloc à ablater ;
**caractérisé en ce que** les paramètres du point d'ablation comprennent en outre un taux de chevauchement, deux points d'ablation adjacents se chevauchent lorsqu'une pluralité de points d'ablation sont prévus entre le premier bloc à ablater et le second bloc à ablater.

2. Procédé de planification automatique pour ablation de tissu selon la revendication 1, dans lequel une formule pour calculer une épaisseur D de la couche d'ablation est la suivante : $D = d \times N$ où d est un diamètre du point d'ablation cylindrique, et N est le taux de chevauchement des deux points d'ablation adjacents.

3. Procédé de planification automatique pour ablation de tissu selon la revendication 1, comprenant en outre :
réobtenir une image 2D lorsque le tissu à ablater se déplace ;
comparer une image 2D précédente avant le déplacement avec l'image 2D réobtenue qui sont dans la même direction ; et
transformer ensemble le modèle 3D et la zone ablatée sur le modèle 3D dans un système de coordonnées de l'image 2D réobtenue.

4. Procédé de planification automatique pour ablation de tissu selon la revendication 1, comprenant en outre :
mettre en œuvre une interpolation pour les images 2D non représentées sur la base de l'image 2D adjacente dans la même direction.

5. Procédé de planification automatique pour ablation de tissu selon la revendication 1, comprenant en outre :
(404), définir une fonction de perte avant de construire le modèle 3D, et affiner le primitif sur l'image 2D à l'aide de la fonction de perte, une formule de la fonction de perte *Loss* étant la suivante : $Loss = 1 - \left({S}_{1}{W}_{1}+{S}_{2}{W}_{2}\right)$
où S1 est une similarité entre un gradient de gris et une valeur moyenne de gris des contours des primitifs sur les deux images 2D adjacentes, S2 est une similarité entre des primitifs sur les deux images 2D adjacentes, et W1 et W2 sont des poids prédéfinis.

6. Procédé de planification automatique pour ablation de tissu selon la revendication 5, dans lequel l'affinement comprend une expansion, une érosion et un décalage de sommet.

7. Procédé de planification automatique pour ablation de tissu selon la revendication 1, comprenant en outre :
(403), mettre en œuvre une interpolation pour les tissus manquants sur l'image 2D après avoir représenté l'image 2D du tissu à ablater.

8. Procédé de planification automatique pour ablation de tissu selon la revendication 1, dans lequel la technique de reconstruction 3D est une technique de kit de segmentation et d'enregistrement Insight (ITK).

9. Dispositif de planification automatique, **caractérisé en ce qu'**il comprend :
une mémoire et un processeur,
dans lequel un programme informatique est stocké dans la mémoire, et le programme informatique est démarré par le processeur pour exécuter le procédé de planification automatique pour ablation de tissu selon l'une quelconque des revendications 1 à 8.
